# EUROPEAN PATENT APPLICATION

(11) **EP 1 518 557 A1**
(43) Date of publication of application: **30.03.2005**
(21) Application number: 03738567.1
(22) Date of filing: 30.06.2003
(51) Int. Cl.: A61K 38/16, A61P 31/18, A61P 37/04, A61P 43/00

(54) **ANTI-HIV AGENT**

(30) Priority: 28.06.2002 JP 2002189534
(71) Applicant: FUSO PHARMACEUTICAL INDUSTRIES LTD., Osaka-shi, Osaka 541-0045 (JP)
(72) Inventor: WAKAMIYA, Nobutaka, Asahikawa-shi, Hokkaido 078-8345 (JP); OHTANI, Katsuki, Asahikawa-shi, Hokkaido 070-8012 (JP); SAKAMOTO, Takashi, Sakurai-shi, Nara 633-0074 (JP); KESHI, Hiroyuki, Osaka-shi, Osaka 558-0042 (JP); KISHI, Yuichiro, Wakayama-shi, Wakayama 640-8324 (JP)
(74) Representative: Gardner, Rebecca
(86) International application number: PCT/JP2003/008259
(87) International publication number: WO 2004/002511

(57) **Abstract**

Disclosed are anti-HIV agents which comprise a mannose binding protein (MBP) as an active component and are useful for effectively inhibiting progress of diseases state in and useful in therapy for individuals infected with human immunodeficiency virus (HIV). Also disclosed are a method for evaluating an anti-HIV activity of MBP comprising the step of culturing HIV infected cells under the presence of MBP.

SEQUENCE LISTING

<110> FUSO PHARMACEUTICAL INDUSTRIES, LTD.

<120> Anti-HIV Agent

<130> 03P451WO

<150> JP 2002-189534
<151> 2002-06-28

<160> 3

<210> 1
<211> 3605
<212> DNA
<213> Homo sapiens

<400> 1
ggtaaatatg tgttcattaa ctgagattaa ccttccctga gttttctcac accaaggtga 60
ggaccatgtc cctgtttcca tcactccctc tccttctcct gagtatggtg gcagcgtctt 120
actcagaaac tgtgacctgt gaggatgccc aaaagacctg ccctgcagtg attgcctgta 180
gctctccagg catcaacggc ttcccaggca aagatgggcg tgatggcacc aagggagaaa 240
agggggaacc aggccaaggg ctcagaggct tacagggccc ccctggaaag ttggggcctc 300
caggaaatcc agggccttct gggtcaccag gaccaaaggg ccaaaaagga gaccctggaa 360
aaagtccgga tggtgatagt agcctggctg cctcagaaag aaaagctctg caaacagaaa 420
tggcacgtat caaaaagtgg ctgaccttct ctctgggcaa acaagttggg aacaagttct 480
tcctgaccaa tggtgaaata atgacctttg aaaaagtgaa ggccttgtgt gtcaagttcc 540
aggcctctgt ggccaccccc aggaatgctg cagagaatgg agccattcag aatctcatca 600
aggaggaagc cttcctgggc atcactgatg agaagacaga agggcagttt gtggatctga 660
caggaaatag actgacctac acaaactgga acgagggtga acccaacaat gctggttctg 720
atgaagattg tgtattgcta ctgaaaaatg gccagtggaa tgacgtcccc tgctccacct 780
cccatctggc cgtctgtgag ttccctatct gaagggtcat atcactcagg ccctccttgt 840
ctttttactg caacccacag gcccacagta tgcttgaaaa gataaattat atcaatttcc 900
tcatatccag tattgttcct tttgtgggca atcactaaaa atgatcacta acagcaccaa 960
caaagcaata atagtagtag tagtagttag cagcagcagt agtagtcatg ctaattatat 1020
aatattttta atatatacta tgaggcccta tcttttgcat cctacattaa ttatctagtt 1080
taattaatct gtaatgcttt cgatagtgtt aacttgctgc agtatgaaaa taagacggat 1140
ttatttttcc atttacaaca aacacctgtg ctctgttgag ccttcctttc tgtttgggta 1200
gagggctccc ctaatgacat caccacagtt taataccaca gctttttacc aagtttcagg 1260
tattaagaaa atctattttg taactttctc tatgaactct gttttctttc taatgagata 1320

ttaaaccatg taaagaacat aaataacaaa tctcaagcaa acagcttcac aaattctcac 1380
acacatacat acctatatac tcactttcta gattaagata tgggacattt ttgactccct 1440
agaagccccg ttataactcc tcctagtact aactcctagg aaaatactat tctgacctcc 1500
atgactgcac agtaatttcg tctgtttata aacattgtat agttggaatc atattgtgtg 1560
taatgttgta tgtcttgctt actcagaatt aagtctgtga gattcattca tgtcatgtgt 1620
acaaaagttt catccttttc attgccatgt agggttccct tatattaata ttcctcagtt 1680
catccattct attgttaata ggcacttaag tggcttccaa tttttggcca tgaggaagag 1740
aacccacgaa cattcctgga cttgtctttt ggtggacatg gtgcactaat ttcactacct 1800
atccaggagt ggaactggta gaggatgagg aaagcatgta ttcagcttta gtagatatta 1860
ccagttttcc taagtgattg tatgaattta tgctcctacc ggcaatgtgt ggcagtccta 1920
gatgctctat gtgcttgtaa aaagtcaatg ttttcagttc tcttgatttt cattattcct 1980
gtggatgtaa agtgatattt ccccatggtt ttaatctgta tttccccaac atgtaataag 2040
gttgaacact tttttatatg cttattgggc acttgggtat cttcttctgt gaagtacccg 2100
ttcacatttt tgtattttgt ttaaattagt tagccaatat ttttcttact gatttttaag 2160
ttatttttac attctgaata tgtccttttt aatgtgtatt acaaatattt tgctagtttt 2220
tgacttgctc ctaatgttga attttgatga acaaaatttc ctaattttga gaaagtctta 2280
tttattcata ttttctttca aaattagtgc tttttgtgtc atgtttaaga aatttttgcc 2340
catcccaaaa tcataagata tttttcatga ttttgaaacc atgaagagat ttttcatgat 2400
tttgaaatca tgaagatatt tttccatttt tttctaatag ttttattaat aaacattcta 2460
tctattcctg gtagaataga tatccacttg agacagcact atgtaggaaa gaccattttt 2520
cctccactga actagggtgg tgcatttttg taagttaggt aactgtatgt gtgtgtgtct 2580
gtttctgggc tgtctattct agtctatttg ttgatgcttg tgtcaaacag tacactatct 2640
taattattgt acatttatag ttgtaactgt agtccagctt tgttcttctt caagtcaaga 2700
tttccatata aatattagaa acagtttctc aatttctaca aaatcctgat gaggtttcta 2760
ctgggaccac attgagtcta tcaatcaact tatgcagaac tggcaactta ctactgaatc 2820
tctaatcaat gttcatcatg tatcgcttca tttaactagg atttctctaa cttaattgct 2880
atgttttgag atttttagtt taaaaacctt gtatatcttg ttttggtggt tttagtgatt 2940
ttaataatat attttaaata tttttctttt tctattgttg tacacagaaa tacagttaag 3000
ttttgtgtgt agtcttacga tgtttagtaa cctcaataag tttatttctt aaatctagta 3060
atttgtagat tcctctggat tttgtatatg catagtcatg taagctgaaa atatggcaat 3120
acttgcttct tcccaattgc tttacctttt ttcttacctt attgcactgg ttagcaaccc 3180
caatacagag accaccagag caggtataga ctcctgaaag acaatataat gaagtgctcc 3240
agtcaggcct atctaaactg gattcacagc tctgtcactt aattgctaca tgatctagag 3300
ccagttactt tgtgtttcag ccatgtattt gcagctgaga gaaaataatc attcttattt 3360
catgaaaatt gtggggatga tgaaataagt taacaccttt aaagtgtgta gtaaagtatc 3420
aggatactat attttaggtc ttaatacaca cagttatgcc gctagataca tgctttttaa 3480
tgagataatg tgatattata cataacacat atcgattttt aaaaattaaa tcaaccttgc 3540
tttgatggaa taaactccat ttagtcacaa aaaaaaaaaa aaaaaaaaaa aaaaaaaaaa 3600
aaaaa 3605

<210> 2
<211> 747
<212> DNA
<213> Homo sapiens

<400> 2
atgtccctgt ttccatcact ccctctcctt ctcctgagta tggtggcagc gtcttactca 60
gaaactgtga cctgtgagga tgcccaaaag acctgccctg cagtgattgc ctgtagctct 120
ccaggcatca acggcttccc aggcaaagat gggcgtgatg gcaccaaggg agaaaagggg 180
gaaccaggcc aagggctcag aggcttacag ggcccccctg gaaagttggg gcctccagga 240
aatccagggc cttctgggtc accaggacca aagggccaaa aaggagaccc tggaaaaagt 300
ccggatggtg atagtagcct ggctgcctca gaaagaaaag ctctgcaaac agaaatggca 360
cgtatcaaaa agtggctgac cttctctctg ggcaaacaag ttgggaacaa gttcttcctg 420
accaatggtg aaataatgac ctttgaaaaa gtgaaggcct tgtgtgtcaa gttccaggcc 480
tctgtggcca cccccaggaa tgctgcagag aatggagcca ttcagaatct catcaaggag 540
gaagccttcc tgggcatcac tgatgagaag acagaagggc agtttgtgga tctgacagga 600
aatagactga cctacacaaa ctggaacgag ggtgaaccca acaatgctgg ttctgatgaa 660
gattgtgtat tgctactgaa aaatggccag tggaatgacg tcccctgctc cacctcccat 720
ctggccgtct gtgagttccc tatctga 747

<210> 3
<211> 248
<212> PRT
<213> Homo sapiens

<400> 3
Met Ser Leu Phe Pro Ser Leu Pro Leu Leu Leu Leu Ser Met Val Ala
1               5                   10                  15
Ala Ser Tyr Ser Glu Thr Val Thr Cys Glu Asp Ala Gln Lys Thr Cys
            20                  25                  30
Pro Ala Val Ile Ala Cys Ser Ser Pro Gly Ile Asn Gly Phe Pro Gly
        35                  40                  45
Lys Asp Gly Arg Asp Gly Thr Lys Gly Glu Lys Gly Glu Pro Gly Gln
    50                  55                  60
Gly Leu Arg Gly Leu Gln Gly Pro Pro Gly Lys Leu Gly Pro Pro Gly
65                  70                  75                  80
Asn Pro Gly Pro Ser Gly Ser Pro Gly Pro Lys Gly Gln Lys Gly Asp
                85                  90                  95
Pro Gly Lys Ser Pro Asp Gly Asp Ser Ser Leu Ala Ala Ser Glu Arg
            100                 105                 110

Lys Ala Leu Gln Thr Glu Met Ala Arg Ile Lys Lys Trp Leu Thr Phe
        115                 120                 125
Ser Leu Gly Lys Gln Val Gly Asn Lys Phe Phe Leu Thr Asn Gly Glu
    130                 135                 140
Ile Met Thr Phe Glu Lys Val Lys Ala Leu Cys Val Lys Phe Gln Ala
145                 150                 155                 160
Ser Val Ala Thr Pro Arg Asn Ala Ala Glu Asn Gly Ala Ile Gln Asn
                165                 170                 175
Leu Ile Lys Glu Glu Ala Phe Leu Gly Ile Thr Asp Glu Lys Thr Glu
            180                 185                 190
Gly Gln Phe Val Asp Leu Thr Gly Asn Arg Leu Thr Tyr Thr Asn Trp
        195                 200                 205
Asn Glu Gly Glu Pro Asn Asn Ala Gly Ser Asp Glu Asp Cys Val Leu
    210                 215                 220
Leu Leu Lys Asn Gly Gln Trp Asn Asp Val Pro Cys Ser Thr Ser His
225                 230                 235                 240
Leu Ala Val Cys Glu Phe Pro Ile
                245

## Description

### [Technical Field]

The present invention relates to novel use of mannose binding protein (hereinafter simply referred to as "MBP"), in particular, to that of MBP as an anti-HIV agent in therapy for infectious diseases caused by human immunodeficiency virus (hereinafter simply referred to as "HIV").

### [Background Art]

MBPs are called as mannan binding proteins, mannan binding lectins, mannose binding lectins and the like and they are belonged to C type lectin, i.e., collectin having, in the molecule thereof, a collagen-like structure and a calcium dependent carbohydrate recognition domain [Kozutsumi, Y. *et al.*, **Biochem. Biophys. Res. Commun.,** 95, pp. 658-664 (1980)].

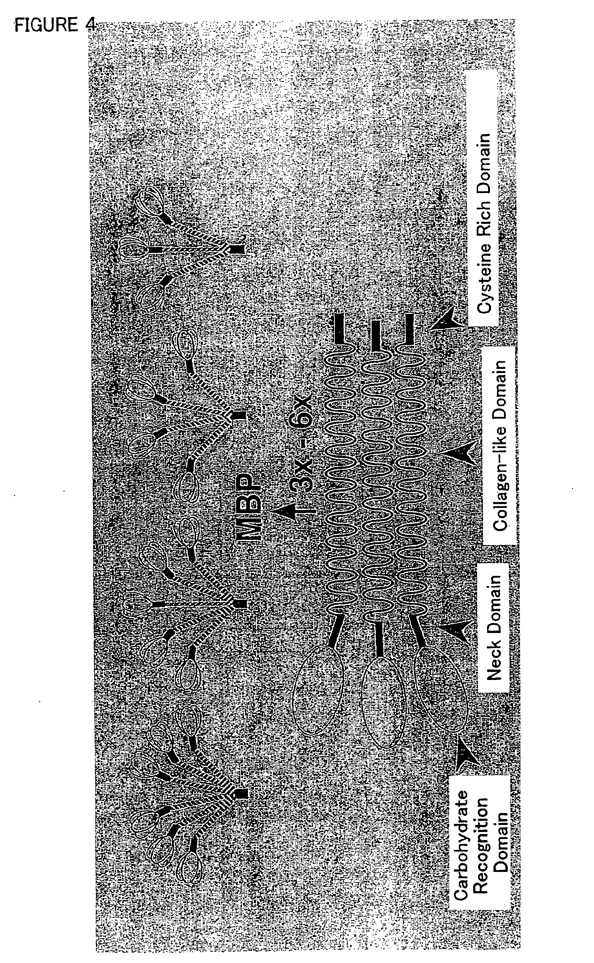

With reference to Fig. 4, MBP is generally consisting of four domains, towards C-terminal from N-terminal region (from left edge to right edge in Fig. 4), of N-terminal domain (cysteine rich domain), collagen-like domain, neck domain and carbohydrate recognition domain (CRD). Then, single subunit structure (trimer) of about 90 kDa to about 99 kDa is formed by binding three polypeptides thereamong with helical structures in both neck domains and collagen-like domains of the single polypeptide having about 30 kDa to about 33 kDa. Further, when two to six of such single subunit structure is bundled, a bouquet-like homo-oligomeric structure is formed.

Prior study on MBP suggested that MBP might participate in initial protection against a microbial infection triggered with an exogenous microorganism by binding it to a carbohydrate chain at the microorganism surface and activating a complement [Holmskov, U. *et al.*, **Immunol**. **Today**, 15, pp. 67-74 (1994) and Turner, M. W. *et al.*, **Immunol**. **Today,** 17, pp. 532-540 (1996)]. Anther reports include that: MBP deficient individuals are liable to get infectious diseases [Summerfield, J. A. *et al.*, **Lancet,** 345, pp. 886-889 (1995)]; there are many MBP deficient individuals among patients suffering from atherosclerosis [Madsen, H.O. *et al*., **Lancet**, 352, pp. 959-960 (1998)]; an MBP gene mutation tends to develop severe malaria [Luty, A. J. *et al.*, **J. Infect. Dis.,** 178, pp. 1221-1224 (1998)]; and the MBP gene mutation renders severe infectious diseases in cystic fibrosis at lung [Garred, P. *et al.*, **J. Clin. Invest.,** 104, pp. 431-437 (1999)].

Moreover, as an investigation directed to use of MBP in a therapeutic medicine, it has been reported that administration of MBP to an MBP deficient patient realized normalization of complement-dependent opsonic activities and leads to the improvement of ready infectivity [Valdimarsson, H. *et al.*, **Scand. J. Immunol.,** 48, pp. 116-123 (1998)]. Additionally, it has also been reported that, when MBP was administered to a patient suffering from cystic fibrosis, clinical symptoms of the patient were stabilized [Garred, P. *et al.*, **Pediatr. Pulmonol.,** 33(3), pp. 201-207 (2002)].

Meanwhile, the number of HIV infected individual increased continuously year after year on a global scale and, in particularly, the developing countries, while number of those suffering from acquired immunodeficiency syndrome (AIDS) have rapidly been increased in such countries. Especially, viruses called as Clade E type in Thailand (Subtype E viruses) have extremely potent infectivity and World Health Organization (WHO) predicted that individuals infected with them would be most highly prevailed then in the world.

Like the situation seen in Europe and the United States, there are many individuals infected with a Subtype B virus in Japan and number of individuals infected with a Subtype E virus seems to be increased in recent years.

For effectively treating HIV infectious diseases, it has been believed to prevent incensement of newly infected individuals or, in other words, to develop a vaccine is an urgent problem. Above all, development of vaccines have actively been performed in the United States, France and the like, then those for Subtype B viruses are currently under investigation, but there is little progress on development any vaccine for Subtype E viruses. NDK strain has been known as a Subtype D virus, while it manifests a fulminant condition and is distributed predominantly in Asia and Central Africa.

Subtype B viruses have been distributed on a global scale as noted above, but the vaccine thereof is currently under investigation and is therefore far from a practical use. Subtype E virus like CRF01_AE has potent infectivity and an expansion of an infection area thereby is expected, while Subtype D viruses include those that manifest a fulminant condition. Accordingly, it has continuously been waited for development of medicine which is effective against viruses belonging to such Subtypes.

AIDS vaccines were studied on live attenuated vaccine containing a viral AIDS virus particle, component vaccine containing a part of the viral particle, recombinant vaccine produced through viral gene recombination, inactivated vaccines containing a protein structure taken from died virus or the like. However, in view of industrially applicability, safety thereof must be considered in addition to efficacy (induction of immunological property) thereof, namely the protective effect against HIV infections.

Currently, multiple drug therapy (highly active anti-retroviral therapy; HAART) has often been used to treat AIDS. Although HAART methodology had been regarded as an epoch-making chemotherapy at the proposing time thereof, administration scheme and kinds of subjected medicine are very complicated at the present in light of the unpreferable factors involved with appearance of drug-resistant viruses, advanced genomic mutation in HIV, adverse effects offered by large dose and long term administration thereof or the like.

HIV is virus having significant genomic diversity and two predominant causes thereof have been suggested.

One element is an increase of diversity due to an error in replication of the HIV genome. Usually, the replication process involves production of DNA by reverse transcriptase from the genomic RNA, followed by incorporation of such DNA into the genome of a host cell. Because the reverse transcriptase lacks 3' → 5' exonuclease activity, it has no editing function for the replicated base sequence. Moreover, because substrate specificity of the reverse transcriptase is low, mutations such as substitution, deletion, insertion, duplication and the like on base is extremely frequently happened at the reverse transcription reaction [Mansky, L. M., **J. Gen. Virol.,** 79, pp. 1337-1345 (1998)].

Additionally, HIV proliferates at a rate of 10⁹⁻¹⁰/day in a living body [Perelson, A. S. *et al*., ***Science,*** 271, pp. 1582-1586 (1996)], thus it is believed that replication of 300 cycles per year has been performed. Therefore, accumulation of mutations is initiated concurrently with HIV infection, thereby further diversity of the genome is arisen.

The other element is an increase of diversity through gene recombination between viruses of different strains is included. Genetic recombination of retroviruses to which HIV belongs has been known to occur with high frequency, in general, through incorporation of two RNA genomes which are homologous with each other into the viral particle, and a mechanism called "forced copy choice'' [Coffin, J. M., **J. Gen. Virol.,** 42, pp. 1-26 (1979)] offered by a template switch function of reverse transcriptase [Jetzt, A. E. *et al*., **J. Virol**., 74, pp. 1234-1240 (2000)]. Therefore, it has been believed that gaining such significant genomic diversity may result in quasispecies of HIV, differentiation of Subtypes, appearance of a recombinant virus, a drug-resistant mutation, a CTL escape mutation and the like.

HIV is generally classified into two types of HIV type 1 (HIV-1) and HIV type 2 (HIV-2) while HIV-1 is further classified into Group M, Group O and Group N, according to the genetic line.

Group M is the most predominant HIV-1 Group, and is classified into 9 Subtypes of A-D, F-H, J and K. The Subtypes A and F are further classified into Sub-Subtypes A, A2 and F1, F2.

Because Subtype E is going to be classified as CRF01_AE at present, Subtype E is hereinafter referred to as "CRFO1_AE'' (*supra*).

HIV-2 is classified into Subtypes A-G [Charneau, P. *et al*., **Virology,** 205, pp. 247-253 (1994); Gurtler, L. G. *et al*., **J. Virol.,** 68, pp. 1581-1585 (1994); Simon, F. *et al.*, **Nat. Med.,** 4, pp. 1032-1037 (1998); Triques, K. *et al.*, **AIDS Res. Hum. Retroviruses,** 16, pp. 139-151 (2000); Robertoson, D. L. *et al*., **Science,** 288, pp. 55-56 (2000); Triques, K. *et al*., **Virology,** 259, pp. 99-109 (1999)].

Moreover, in the classification of recombinant viruses of HIV, recombinant epidemic forms (CRFs: CRF01-CRF12) strains, unclassified recombinant forms (URFs: URFs A/C, URFs A/D, URFs B/E, URFs B/C) viruses, recombinant viruses of which classification is unknown (MAL strain), recombinant viruses between Groups M/O and the like have been reported [Carr,J. K. *et al.*, **Virology**, 247, pp. 22-31 (1998); Motomura, K, *et al.,* **AIDS Res. Hum. Retroviruses,** 16, pp. 1831-1843 (2000); Peeters, M. *et al.,* **J. Virol.,** 73, pp. 7368-7375 (1999); Takehisa, J. *et al*., **J. Virol.,** 73, pp. 6810-6820 (1999); Yutaka Takebe, **Journal of the Japanese Society for Virology,** 50(2), pp. 123-138 (2001)].

Glycoproteins of HIV include gp120 which is an envelope glycoprotein, and gp41 which is a transmembrane glycoprotein. These glycoproteins are generated from gp160 which is encoded by a gene referred to as "env" having a viral genomic form through cleavage with protease of the host [Hallenberger, S. *et al*., **Nature,** 360, pp. 358-361 (1992)].

Because there are 24 sites as N-bound carbohydrate chain binding site in gp120 [Leonard, C. K. *et al*., **J. Biol. Chem.,** 265, pp. 10373-10382 (1990)], wherein about half of gp120 is the carbohydrate chains [Allans, J. S. *et al*., **Science,** 228, pp. 1091-1094 (1985)], HIV may be referred to as a virus covered by carbohydrate chains. It was demonstrated by various experiments that the carbohydrate chains of gp120 are necessary for infection of target cells with HIV [Fennie, C. *et al.,* **J. Virol.,** 63, pp. 639-646 (1989); Matthews, T. J. *et al.*, **Proc. Natl. Acad. Sci. USA,** 84, pp. 5424-5428 (1987); Montefiori, D. C. *et al.*, **Proc. Natl. Acad. Sci. USA,** 85, pp. 9248-9252 (1988); Pal, R. *et al*., **Proc. Natl. Acad. Sci. USA,** 86, pp. 3384-3388 (1989)].

Meanwhile, as a mechanism triggered when a cell is infected with an HIV-1 particle, a series of activities have been assumed in which gp120 is first bound to CD4 on a cell membrane, followed by binding of a V3 loop of gp120 to a coreceptor (chemokine receptor), and invasion of gp41 into the cell membrane to cause membrane fusion. A variety of chemokine receptors have been reported so far, including for example, CCR5 (coreceptor of macrophage-tropic viruses), CXCR4 (coreceptor of T cell-tropic viruses), CCR1, CCR2b, CCR3, CCR4, CCR8, CCR9, CXCR2, CXCR5, CXCR6/STRL33, CX3CR1 and the like. As a methodology to classify HIV, one classification is performed on the basis of these chemokine receptors, in addition to the process relied on the genetic line as described above. For example, viruses that infect via CCR5 as a coreceptor are classified into CCR5 (may be also referred to merely as R5)-tropic viruses, viruses that infect via CXCR4 as a coreceptor are classified into CXCR4 (may be also referred to merely as X4)-tropic viruses, and in a similar manner, HIV may be classified into CCR1-tropic viruses, CCR2b-tropic viruses and the like. Among these, in particular, R5-tropic viruses are macrophage-tropic viruses, and X4-tropic viruses are classified into T cell-tropic viruses. Moreover, R5X4-tropic viruses are classified into viruses that exhibit tropism towards both macrophages and T cell.

In connection with the relationship between MBP and HIV, it was reported that risk of an infection with HIV is high in humans with a homo gene mutation of MBP, and the shorter survival time period following diagnosis of AIDS is observed [Garred, P. *et al.*, **Lancet,** 349, pp. 236-240 (1997)]. However, it was thereafter reported that the group with a gene mutation is more resistant to progress to AIDS, to the contrary [Mass, J. *et al.,* **AIDS,** 12, pp. 2275-2280 (1998)], and it was also reported that a gene mutation is unrelated to prognosis of AIDS [McBride, M.0. *et al.*, **Int. J. STD. AIDS**., 9, pp. 683-688 (1998)]. Additionally, it was reported that MBP binds to gp120 [Larkin, M. *et al.*, **AIDS,** 3, pp. 793-798 (1989); Mizuochi, T. *et al.,* **J. Biol. Chem.,** 264, pp. 13834-13839 (1989); Saifuddin, M. *et al.*, **J. Gen. Virol.,** 81, pp. 949-955 (2000)].

However, the presence of anti-HIV activity of MBP as well as the presence of a proliferation suppressive activity on HIV by MBP was completely uncertain. This fact indicated that such function have not been demonstrated at all, though they are the most important factors for the determination of usefulness upon utilization of MBP as an anti-HIV agent.

Moreover, because there is not any evaluation system which enables evaluation of an anti-HIV activity of MBP, development of an anti-HIV agent containing MBP is not advanced yet substantially.

Furthermore, problems involving appearance of drug resistant viruses, advanced genomic disease states of HIV, adverse effects caused by long-term administration and a large amount administration of medication, which are concerned upon therapy of HIV with an agent, remain unresolved. Also, there is no substantial development of a vaccine, in particular, that on Subtype E.

In the meantime, it has been elucidated that R5-tropic viruses are hard to be neutralized by a neutralizing antibody, and closely related to progress of a disease state. Above all, because a therapeutic process against an R5-tropic virus during the early stage of an infection has been believed to significantly affect the prognosis of the patient, an accurate care at an earlier stage of an infection towards R5-tropic viruses is necessary. Further, because almost of HIV that infect and spread are CCR5-tropic viruses, CCR5 is attracted attention as a target for prophylactic and suppression of an infection and spreading. On the other hand, it is known that as the disease state go towards the later stage according to the clinical course, transition from a CCR5-tropic virus to a CXCR4-tropic virus proceeds, and in the later stage, the presence of a CXCR4-tropic virus becomes prominent.

Elucidation of the presence of such chemokine receptors was achieved quite recently, development of therapeutic drugs targeted to these chemokine receptors are therefore still in the basic research step. Although development of inhibitors of these chemokine receptors have been also carried out, serious problems to be concerned remain unresolved, such as appearance of the CXCR4-tropic virus accelerated when a CCR5 inhibitor is administered, resulting in a concern it may promote progress of the disease state.

Therefore, clinicians long for development of safe agents which can obviate a series of problems as described above and exhibit an anti-HIV activity irrespective of various factors such as Subtype of HIV virus, tropism towards a chemokine receptor, as well as tropism towards macrophages or T cell.

Among all, development of agents that exhibit an anti-viral activity against Subtype B viruses, CRF01_AE and Subtype D viruses, as well as development of agents that exhibit an anti-viral activity against CCR5-tropic viruses, CXCR4-tropic viruses and CCR5/CXCR4-tropic viruses have been desired. In addition, development of agents that exhibit an anti-viral activity against macrophage-tropic viruses, T cell-tropic viruses, and macrophage/T cell-tropic viruses has been also desired similarly.

### [Disclosure of The Invention]

The present invention was established in view of the aforenoted problems in the prior art, in particular, to realize for the first time an evaluation system which enables quantification of an anti-HIV activity of MBP. Furthermore, use of MBP as an anti-HIV agent was also realized for the first time through verifying an anti-HIV activity of MBP, i.e., HIV proliferation suppressive activity by utilizing such evaluation system. Moreover, the present inventors demonstrated that MBP exhibits an anti-HIV activity against not only Subtype E HIV, but also HIV belonging to other clades (Subtypes).

Accordingly, a merit of the present invention is an anti-HIV agent which comprises MBP as an active ingredient. The anti-HIV agent containing MBP as an active component is advantageous because the target of MBP is a carbohydrate chain, thereby, it is not affected well by the appearance of MBP resistant strain due to HIV genomic mutation. Moreover, because MBP is a substance that usually exists in a living body, it is advantageous in no adverse effect found for compounds used in conventional chemotherapies.

Additionally, according to another embodiment of the present invention, a method for evaluating an anti-HIV activity, i.e., a method for evaluating an anti-HIV activity offered by MBP is also provided which comprises the steps of:
(1) culturing HIV infected cells prepared by exposing target cells to HIV;
(2) preparing clean cells by washing the infected cells;
(3) culturing the clean cells in the presence of MBP; and
(4) detecting p24 protein from HIV in the culture supernatant.

Further, according to yet another embodiment of the present invention, other evaluation method for an anti-HIV activity, i.e., an evaluation method of an anti-HIV activity offered by MBP is also provided which comprises the steps of:
(a) culturing a first mixed system including HIV and MBP;
(b) culturing a second mixed system including target cells and MBP;
(c) preparing infected cells by combining said first mixed system and second mixed system;
(d) culturing the infected cells;
(e) preparing clean cells by washing the infected cells;
(f) culturing the clean cells; and
(g) detecting p24 protein from HIV in the culture supernatant.

These evaluation methods enable one to easily confirm and verify, in an objective manner, an anti-HIV activity which is possessed potentially by MBP.

### [Brief Description of The Drawings]

Fig. 1 is a graph showing a correlation between a recombinant mannose binding protein (rMBP) and an HIV activity in HIV-infected cells NDK/M8166.
Fig. 2 is a graph showing a correlation between a recombinant mannose binding protein (rMBP) and an HIV activity in HIV-infected cells LP65/M8166.
Fig. 3 is a graph showing a correlation between a natural mannose binding protein (nMBP) and an HIV activity in HIV-infected cells NDK/M8166.
Fig. 4 is a schematic view showing the structure of mannose binding protein.

### [Best Mode for Carrying Out The Invention]

The anti-HIV agent according to the present invention makes use of HIV proliferation suppressive activity, for example, HIV neutralizing activity, HIV budding suppressive activity and the like, respectively offered by MBP employed as the active component, therefore, the agent is useful for therapy in and to inhibit progress of the disease state on AIDS patients and HIV-infected individuals.

Furthermore, MBP as an active component of the anti-HIV agent according to the present invention is permitted to use in anyone as long as MBP offers certain HIV proliferation suppressive activity, irrespective of natural product form and synthetic product form (including recombinant products). In particular, MBP including one isolated and purified from a human serum and the other genetically secreted from an animal cell, preferably, Chinese Hamster Ovary (CHO) cell (hereinafter simply referred to as "CHO cell'') are suitably used in the present invention.

Recombinant human mannan binding protein (hereinafter simply referred to as "rhMBP") which is one of the applicable MBP in the present invention can be produced, for example, with reference to International Publication Number WO 99/37676, according to the serial steps of (i) constructing the expression vector pNOW1-hMBP by inserting, into the plasmid pNOW1, a polynucleotide consisting of continuous 747 nucleotides (SEQ ID NO: 2) corresponding to from 66 bp to 812 bp of the base sequence of the cDNA (SEQ ID NO: 1) in a natural human mannan binding protein (hereinafter, simply referred to as "nhMBP''), (ii) preparing a transformant by introducing the expression vector pNOW1-hMBP into CHO cells lacking dihydrofolate reductase (dhfr⁻), (iii) preparing neomycin resistant cells by culturing the transformant in a culture medium containing neomycin, (iv) preparing methotrexate resistant cells by culturing the selected neomycin resistant cells in a culture medium containing methotrexate (MTX), and (v) collecting rhMBP from the selected methotrexate resistant cells.

The producing method of the present invention is explained in detail as follows.

First of all, the expression vector pNOW1-hMBP is constructed. The amino acids which constitute nhMBP had been previously analyzed and reported by Herman et al., [Sastry et al., "The human mannose-binding protein gene. Exon structure reveals its evolutionary relationship to a human pulmonary surfactant gene and localization to chromosome 10", **J. Exp. Med**. 170(4), pp. 1175-1189 (1989)]. The amino acid sequence which constitutes nhMBP is set out in SEQ ID NO: 3.

Taking such sequence information into consideration, base sequence corresponding to from the initiation codon to stop codon in nhMBP is amplified from a human liver cDNA library (Clonetech), and the amplified cDNA of nhMBP is digested with a restriction enzyme to obtain a polynucleotide consisting of the continuous 747 nucleotides (SEQ ID NO: 2) corresponding to from 66 bp to 812 bp in cDNA of nhMBP to generate an insert. The expression vector pNOW1 is then digested with a restriction enzyme, and such insert is inserted between pCMV and BGP poly A with a DNA ligation kit (Takara Shuzo). The expression vector so obtained is designated as plasmid pNOW1-hMBP.

Selection of the expression clone is then performed. Scheme to introduce the expression vector pNOW1-hMBP into CHO cells lacking dihydrofolate reductase (dhfr⁻) is performed as follows. IMDM medium (GIBCO) supplemented with fetal calf serum is prepared and is mixed with (dhfr⁻) DG44 CHO cell strain, followed by 24 hrs cultivation under the condition of at 37°C, in 5% carbon dioxide gas. The culture supernatant is discarded and IMDM is added instead to the remained culture wherein such IMDM is supplemented with FCS containing a solution previously prepared by mixing the expression vector pNOW1-hMBP with a lipofectin solution. After further adding thereto hypoxanthine (GIBCO) and thymidine (GIBCO), culture is performed to realize introduction of the expression vector pNOW1-hMBP into dhfr- host CHO cells. Thereafter, the culture supernatant is discarded and IMDM is added instead to the remained culture wherein such IMDM is supplemented with FCS, hypoxanthine and thymidine, followed by additional culture.

In order to take neomycin (G418) resistant CHO cells, after culturing the cells with the expression vector pNOW1-hMBP, a trypsin treatment is performed, and the cells are suspended in IMDM supplemented with FCS containing neomycin (G418). This suspension is then poured onto a microplate, and neomycin resistant cells (clones) are appeared by two weeks cultivation under the condition of at 37°C in 5% carbon dioxide gas (CO₂).

Several clones are selected among the clones verified for the production of rhMBP, and each clone is cultured. Each culture supernatant is discarded and IMDM is added instead to the remained culture wherein such IMDM is supplemented with FCS containing the same composition as noted above, followed by 4 days culture. The culture supernatant is then collected. Amount of produced rhMBP in the collected culture supernatant is determined. The amount of the produced rhMBP can be determined pursuant to the method of Suzuki *et al*., [Y. Suzuki, *et al.*, "Characterization of Recombinant Bovine Conglutinin Expressed in a Mammalian Cell", **Biochem. Biophys. Res. Commun**., 238, pp. 856-863 (1997)] employing nhMBP as a control, an anti-rabbit polyclonal antibody to the carbohydrate recognition domain (CRD) and neck domain of collectin (expressed in *E. coli),* and nhMBP (subject for determination).

In order to take methotrexate resistant CHO cells, rhMBP producing clone is subjected to further passage culture to allow stabilization, and gene amplification is then performed after addition of methotrexate to the medium at a low concentration. As the first step, each selected cell clone is added to seed them into IMDM supplemented with 10% dialyzed FCS (JRH Biosciences) containing methotrexate and neomycin (G418). Methotrexate resistant cells (clones) are appeared by 2 weeks cultivation under the condition of at 37°C, in 5% carbon dioxide gas (CO₂). When productivity of rhMBP in these methotrexate resistant clones is confirmed, high production level is verified.

Some clones are optionally selected from these clones, and each selected clone is seeded and is cultured for two weeks. The culture supernatant is discarded, and IMDM is added to the remained culture wherein IMDM is supplemented with FCS containing the same composition as described above (containing methotrexate and neomycin (G418)). After 4 days cultivation, the culture supernatant is collected to determine the production level of rhMBP.

In order to purify rhMBP, a clone which showed the highest production activity among the collected clones is seeded and is cultivated. The culture supernatant is then discarded and CHO-S-SFM II medium (vitamin C is added thereto to give 100 mM when addition of vitamin C is desired) is added to the remained culture wherein the medium contains methotrexate and neomycin (G418). Cultivation is then continued for 4 days. The culture supernatant is collected, dialyzed against TBS (prepared from TBS powder (Takara Shuzo)) and successively against TBSC (5 mM CaCl₂, TBS). Purification is performed with mannan-agarose (SIGMA). Specifically, a column (Column PD-10, Empty, Pharmacia) is loaded with mannan-agarose, is flowed thereinto with the dialyzed culture liquid, is washed with TBSC, and is eluted with TBSE (10 mM EDTA, TBS). After the elution, 1M CaCl₂ is added to realize the final concentration of 15 mM. Then, the mixture is applied to mannan-agarose again, is washed with TBSC, and is eluted with TBS containing 100 mM mannose. Thereafter, dialysis against TBSC is performed again to produce the purified rhMBP.

The rhMBP so purified is applied to gel filtration chromatography and exhibits a specific peak of absorbance at 280 nm at a molecular weight of 1,000 to 1,300 kDa, particularly, at a molecular weight of 1,150 kDa. Specific peak is also exhibited at a molecular weight of 200 to 400 kDa, particularly at a molecular weight of 300 kDa. Gel filtration chromatographic analysis of rhMBP is performed using 20 mM Tris-HC1 (pH 8.0), 0.15 mM NaCl, 5 mM EDTA under the condition of at a flow rate of 0.5 ml/min, with Superose 6 HR10/30 (φ 10mm × 300mm length; Pharmacia) . Then, 40µg of rhMBP is loaded onto this column, and the absorbance of 280 nm is determined.

When an artificial rhMBP synthesized according to the method disclosed in International Publication No. WO 99/37676 is used as an active component in the anti-HIV agent of the present invention, the purified rhMBP as noted previously is preferably used. rhMBP fraction appearing around a molecular weight of 1,000 to 1,300 kDa as well as the other rhMBP fraction appearing around a molecular weight of 200 to 400 kDa respectively determined with the absorbance at 280 nm by applying the purified rhMBP described above onto a gel filtration chromatography. Otherwise, the purified rhMBP including all of these fractions may also be used.

Anti-HIV agent of the present invention has effective function on any type of HIV strains, but obviously from the following Examples, remarkable anti-HIV activity have been offered against Subtype B of HIV-1 Group M, as well as Subtype D of HIV-1 Group M, and recombinant epidemic strain, in particular, CRF01_AE strain.

Anti-HIV agent of the present invention also offered a remarkable anti-HIV activity against CCR5-tropic viruses, CXCR4-tropic viruses, and CCRS/CXCR4-tropic viruses. Further, the anti-HIV agent of the present invention offered a similar remarkable anti-HIV activity against macrophage-tropic viruses, T cell-tropic viruses, and macrophage/T cell-tropic viruses.

When the anti-HIV agent of the present invention is used as a therapeutic agent for AIDS or a progress suppressive agent against a disease state of an HIV infection, a dosage form and an administration route (intravenous administration or the like) should be selected in light of the better incorporation of protein into a living body. For example, as a method for administering a pharmaceutical composition of the present invention, besides the intravenous administration, transmucosal administration, transdermal administration intramuscular administration, subcutaneous administration, endorectal administration, oral administration and the like can be selected, and the agent form can be changed optionally according to the method of the administration. Formulations for intravenous administration are described below, but the dosage form to be used in the present invention is not limited thereto. Various formulation types which are used generally in the pharmaceutical formulation can be utilized.

It has been reported that, on Japanese subjects (479 samples), when gene mutation of MBP and the MBP concentration in blood were studied, a gene mutation of MBP was found only in codon 54 (GGC →GAC), whilst the proportion of each genotype was 70.8% for Wild/Wild, 22.5% for Wild/mutant, and 6.7% for mutant/ mutant. Also, it has been reported that the MBP concentration in blood was 0.18 to 4.35 µg/ml (average: 1.26 µg/ml) for Wild/Wild, 0.00 to 0.80 µg/ml (average: 0.23 µg/ml) for Wild/mutant, and 0.00 to 0.20 µg/ml (average: 0.04 µg/ml) for mutant/mutant [Hiroyuki Keshi *et al*., **IGAKU NO AYUMI (Journal of Clinical and Experimental Medicine),** 194(12), pp. 957-958 (2000)].

When the anti-HIV agent of the present invention is used as a therapeutic agent for AIDS or a progress suppressive agent on a disease state of an HIV infection, the intravenous administration amount of the anti-HIV agent of the present invention may be determined by referring to MBP concentration in blood analyzed with some methodology like ELISA, such that the MBP concentration in blood adjusts firstly to from about 1 µg/ml to about 1.5 µg/ml, when the amount of the produced MBP is lowered owing to liver damage such as hepatitis C, or when the concentration in blood is low due to a gene mutation, in view of the facts that the MBP concentration in blood of a healthy human is from about 1 µg/ml to about 1.5 µg/ml. It is necessary to determine the effective MBP concentration in blood by periodically monitoring the amount of HIV-RNA and the number of CD4-positive cells and taking into the consideration the relationship between the monitoring results and the amount of MBP administered. When MBP concentration in blood is within a normal value range, effective MBP concentration in blood may be determined by gradually elevating the concentration thereof in blood (for example, from about 1.5 µg/ml to about 5.0 µg/ml), periodically monitoring the amount of HIV-RNA and the number of CD4-positive cells, and taking into the consideration the relationship between the monitoring results and the amount of MBP administered.

The effective MBP concentration in blood shall not be constant because it is depended on inborn MBP concentration in blood, but such concentration is preferably adjusted to from about 1.0 µg/ml to about 50 µg/ml, and more preferably from about 1.5 µg/ml to about 10 µg/ml. MBP concentration in blood may deviate from these ranges at immediately before or after an administration. MBP concentration in blood will also be reduced due to gene mutation, administration amount of MBP should therefore also be determined in consideration of such gene mutation.

Further, additives such as solvent, excipient, coating agent, base, binder, lubricant, disintegrant, solubilizing agent, suspending agent, thickening agent, emulsifying agent, stabilizing agent, buffering agent, isotonizing agent, soothing agent, preservative, flavoring agent, aromatizing agent, coloring agent and the like may be added as raw materials for pharmaceutical formulations according to the dosage form thereof (known form like formulation for oral administration, injectable formulation, suppository or the like).

Specific examples of such additives are illustrated below, but are not limited thereto.
- Solvent:: purified water, water for injection, physiological
saline, peanut oil, ethanol, glycerin.
- Excipient:: starches, lactose, glucose, sucrose, crystalline
cellulose, calcium sulfate, calcium carbonate, talc, titanium oxide, trehalose, xylitol.
- Coating agent:: sucrose, gelatin, cellulose acetate phthalate, and the above listed polymeric excipient.
- Base:: vaseline, vegetable oil, macrogol, base for oil in water emulsion, base for water in oil emulsion.
- Binder:: starch and derivatives thereof, cellulose and derivatives thereof, gelatin, sodium alginate, natural polymer compounds like tragacanth, gum arabic and so on, synthetic polymer compounds like polyvinylpyrrolidone and so on, dextrin, hydroxypropyl starch.
- Lubricant:: stearic acid and salts thereof, talc, waxes, wheat starch, macrogol, hydrogenated vegetable oil, sucrose fatty acid ester, polyethylene glycol.
- Disintegrant:: starch and derivatives thereof, agar, gelatin
powder, sodium hydrogen carbonate, cellulose and derivatives thereof, carmellose calcium, hydroxypropyl starch, carboxymethylcellulose and salts/crosslinked forms thereof, low substituted hydroxypropylcellulose.
- Solubilizing agent:: cyclodextrin, ethanol, propylene glycol, polyethylene glycol.
- Suspending agent:: gum arabic, tragacanth, sodium alginate, aluminum monostearate, citric acid, various types of surfactants.
- Thickening agent:: carmellose sodium, polyvinylpyrrolidone, methylcellulose, hydroxypropylmethylcellulose, polyvinyl alcohol, tragacanth, gum arabic, sodium alginate.
- Emulsifying agent:: gum arabic, cholesterol, tragacanth, methylcellulose, various types of surfactants, lecithin.
- Stabilizing agent:: sodium hydrogen sulfite, ascorbic acid, tocopherol, chelating agent, inert gas, reducing substances.
- Buffering agent:: dibasic sodium phosphate, sodium acetate, boric acid.
- Isotonizing agent:: sodium chloride, glucose.
- Soothing agent:: procaine hydrochloride, lidocaine, benzyl alcohol.
- Preservative:: benzoic acid and salts thereof, parahydroxybenzoate esters, Chlorobutanol, inverted soap, benzyl alcohol, phenol, thimerosal.
- Flavoring agent:: sucrose, saccharin, glycyrrhiza extract, sorbitol, xylitol, glycerin.
- Aromatizing agent:: orange peel tincture, rose oil.
- Coloring agent:: water soluble edible dye, lake dye.

Beside the ingredients listed above, the anti-HIV agent according to the present invention may contain a pharmaceutically acceptable salt. Examples of the pharmaceutically acceptable salt(s) include, for example, salts with an inorganic base, an organic base or the like, acid addition salts with an inorganic acid, an organic acid, a basic or acidic amino acid or the like. Specific examples of such salt(s) are illustrated below, but are not limited thereto.
- Inorganic base:: alkali metals such as sodium, potassium and the like, alkaline earth metals such as calcium, magnesium and the like, aluminum, ammonium and the like.
- Organic base:: primary amines like ethanolamine and so on, secondary amines like diethylamine, diethanolamine, dicyclohexylamine, N,N'-dibenzylethylenediamine and so on, tertiary amines like trimethylamine, triethylamine, pyridine, picoline, triethanolamine and so on.
- Inorganic acid:: hydrochloric acid, hydrobromic acid, nitric acid, sulfuric acid, phosphoric acid.
- Organic acid:: formic acid, acetic acid, lactic acid, trifluoroacetic acid, fumaric acid, oxalic acid, tartaric acid, maleic acid, benzoic acid, citric acid, succinic acid, malic acid, methanesulfonic acid, ethanesulfonic acid, benzenesulfonic acid, p-toluenesulfonic acid.
- Basic amino acid:: arginine, lysine, ornithine.
- Acidic amino acid:: aspartic acid, glutamic acid.

### [Examples]

The present invention is specifically illustrated below as Examples, but, as a matter of course, the present invention should not be limited by the disclosure of these Examples.

Although the term "anti-HIV activity'' referred to in the following Examples involves not only HIV neutralizing activity, but also HIV budding suppressive activity, it is simply noted as "neutralizing activity'' as a matter of convenience.

HIV strains used in Examples 1 and 2 are summarized in Table 1.

**Table 1**

| Viral strain | Tropism | Chemokine receptor | Subtype |
|---|---|---|---|
| 92Th014 | Macrophage | CCR5 | B |
| JRCSF | Macrophage | CCR5 | B |
| LP65 | Macrophage/T cell | CCR5/CXCR4 | E |
| NDK | Macrophage/T cell | CCR5/CXCR4 | D |

### Example 1

Anti-HIV activity by MBP was examined in this Example.

First of all, as HIV strains, HIV-NDK experimental strain (Subtype D; hereinafter simply referred to as "NDK"), and recombinant epidemic strain CRF01_AE (clinical isolate, hereinafter simply referred to as "LP65'') were provided.

Then, human peripheral blood mononuclear leukocyte (human PBMC; hereinafter simply referred to as "PBMC") which has been subjected to blastogenesis with phytohemagglutinin (PHA) as well as established cell M8166 of T cell line (hereinafter simply referred to as "M8166") were infected respectively with these HIV strains.

Both NDK and M8166 are strains available from AIDS Research and Reference Regent Program of National Institutes of Health (NIH).

Both nhMBP purified from human blood and rhMBP synthesized by the method disclosed in International Publication No. WO 99/37676 were used as MBP.

NDK which is a fulminate virus was added to PBMC and M8166 respectively, and these were cultivated at 37°C for 1 hour to allow an infection. Infected strains so produced are designated respectively as NDK/PBMC and NDK/M8166. Further, LP65 was added to PBMC and M8166 respectively, and these were cultivated at 37°C for 1 hour to allow HIV infection. Both NDK and LP65 were added, with an amount corresponding to 100 TCID₅₀ viruses, to cells of 5 × 10⁶ cells/ml. Infected cells so produced were designated respectively as "LP65/PBMC" and "LP65/M8166".

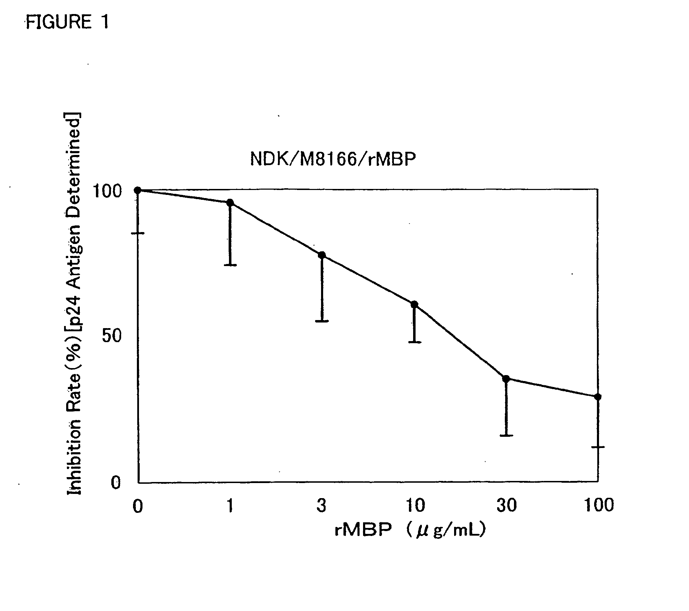

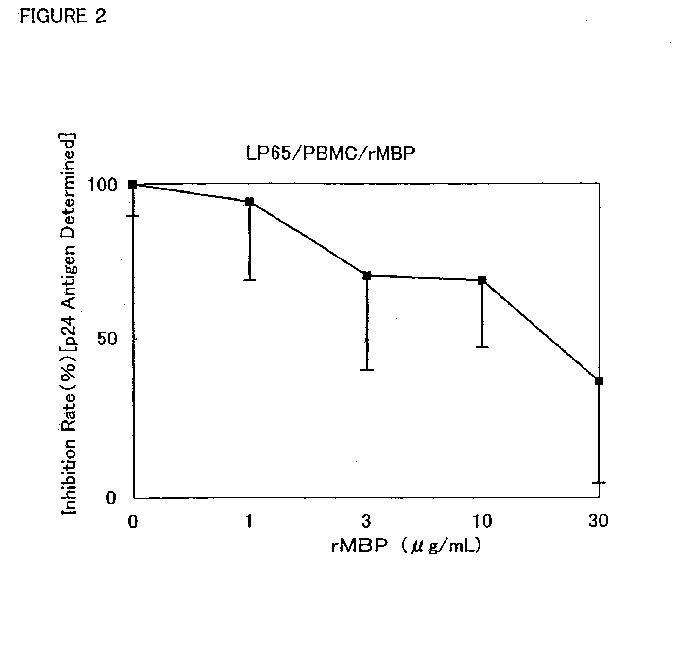

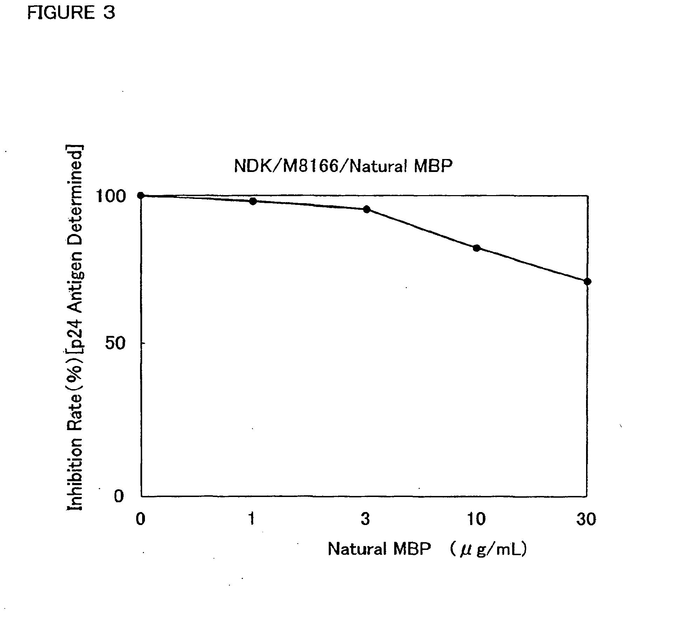

Infected cells were washed twice with PBS. With regard to four kinds of virus-infected cells (NDK/PBMC, NDK/M8166, LP65/PBMC, LP65/M8166), nhMBP or rhMBP is added thereto at a concentration of from 1 to 100 µg/ml (Fig. 1), or at a concentration of from 1 to 30 µg/ml (Fig. 2 and Fig. 3). Cell density at that time was 1 × 10⁶ cells/ml per 200 µl (i.e., 2 × 10⁵ cells/200 µl). In other words, in 1/5 scale system of 200 µl, cells were placed in the concentration of 1-100 µg MBP/1 × 10⁶ cells/ml or of 1-30 µg MBP/1 × 10⁶ cells/ml, followed by one week cultivation in a medium supplemented with a human serum at 37°C, 5% carbon dioxide gas. As an indicator on amount of viruses after the cultivation, an amount of p24 antigen from HIV in the culture supernatant was determined with ELISA using a fully automated chemiluminescent enzyme immunoassay system (Lumipulse f: Fujirebio), and was subjected to the comparison with the control group without MBP.

Consequently, as shown in the graph of Fig. 3, suppression of the HIV amount in the nhMBP added group (HIV proliferation suppression) was observed. In addition, obviously from the graph of Fig. 1, with regard to NDK/M8166, the amount of p24 antigen in the culture supernatant at the rhMBP concentration of 10 µg/ml-30 *µ*g/ml was suppressed immediately after the culture to about 50% of the control group without rhMBP. Further, obviously from the graph of Fig. 2, rhMBP offered anti-HIV effect (HIV proliferation suppressive effect) in the clinical isolate LP65 (recombinant epidemic strain of HIV-1, CRF01_AE) at IC₅₀ concentration of about 10 *µ*g/ml wherein LP50 is neutralized in a concentration dependent manner, in other words, there were anti-HIV activity (neutralizing activity) irrespective of Subtypes.

There was not any remarkable proliferation suppression and any cell toxicity against the cells in the tried MBP concentration range on both nhMBP and rhMBP. According to the present experimental system, HIV budding suppressive activity was suggested, in the other words, it seems that release of HIV from the infected cells were duly suppressed.

Foregoing results revealed that the anti-HIV agent of the present invention offered the anti-HIV activity against both Subtype E HIV and Subtype D HIV. Similarly, the results also revealed that the anti-HIV agent of the present invention offered the anti-HIV activity against CCR5/CXCR4-tropic viruses and Macrophage/T cell-tropic viruses.

### Example 2

92Th014 Laboratory Strain (Subtype B) and JRCSF Laboratory Strain (Subtype B) were provided as HIV strains. Any of these Laboratory Strain is available from AIDS Research and Reference Regent Program of National Institutes of Health. PBMC, nhMBP and rhMBP were those noted in Example 1.

First of all, 50 µl of viral solution corresponding to 100 TCID₅₀ titer, and 50 µl of nhMBP or rhMBP solution at their final concentration of 2, 6, 20, 60 µg/ml were mixed. This mixed solution was then cultivated at 37°C in the presence of 5% carbon dioxide gas for 1 hour to prepare a first mixed system containing nhMBP or rhMBP at the final concentration of 1, 3, 10, 30 µg/ml.

Simultaneously, concentration of PBMC was adjusted to 2 × 10⁵ cells/50 µl, and they were mixed with 50 µl of nhMBP or rhMBP solution at their final concentration of 2, 6, 20, 60 µg/ml. This mixed solution was then cultivated at 37°C in the presence of 5% carbon dioxide gas for 1 hour to prepare a second mixed system containing nhMBP or rhMBP at the concentration of 1, 3, 10, 30 µg/ml.

MBP concentrations (final concentration) in the first mixed system solution and the second mixed system solution is evenly adjusted to be any of 1, 3, 10 and 30 µg/ml, then the both solution were combined and cultured all day and night at 37°C. Unreacted MBP and viruses were removed by washing them. Fresh nhMBP or rhMBP was added to the culture to realize the starting MBP concentration and it was cultivated for 7 days in an RPMI medium (containing 20 units/ml Interleukin-2, 50 units/ml penicillin and 50 units/ml streptomycin) supplemented with 10% FCS. As an indicator on amount of viruses after the cultivation, an amount of p24 antigen from HIV in the culture supernatant was determined with ELISA using a fully automated chemiluminescent enzyme immunoassay system (Lumipulse f: Fujirebio), and was subjected to the comparison with the control group without MBP.

There were significant anti-HIV activity (HIV proliferation suppressive activity) against any virus of JRCSF and 92Th014 which are wild type viruses. On JRCSF, necessary amount of MBP to halve the amount of p24 antigen (50% suppression concentration) was 0.19 µg or less and 2.74 µg or less for nhMBP and rhMBP respectively. Similarly, on p2Th014, it was 7 µg or less and 1.57 µg or less for nhMBP and rhMBP respectively. These results clearly indicated that MBP also offered a remarkable anti-HIV activity (neutralizing activity) against Wild Type Subtype B virus strains.

Results of this Example also revealed that the anti-HIV agent of the present invention also offered an anti-HIV activity against CCR5-tropic viruses and Macrophage-tropic viruses. Since anti-HIV activity against a CCR5-tropic virus by MBP was directly demonstrated in this Example, it was proven that MBP can be effectively used for not only therapy for an earlier stage of HIV infection, but also prophylactic therapy or suppression of infection and infection spreading. Further, this Example directly demonstrated that the anti-HIV agent of the present invention also offered the anti-HIV activity against the CCR5/CXCR4-tropic viruses, it was therefore revealed that the anti-HIV agent of the present invention is effective not only in the earlier stage of HIV infection, but also in the disease states wherein clinical course is progressed (i.e., not only HIV infected individuals but also HIV patients).

Results in Examples 1-2 taught that, beside anti-HIV activity against CCR5/CXCR4-tropic viruses, MBP further offered similarly anti-HIV activity against CCR5-tropic viruses, MBP is therefore believed to offer the similar anti-HIV activity against CXCR4-tropic viruses. Likewise, beside the anti-HIV activity against Macrophage/T cell-tropic viruses, MBP further offered the similar anti-HIV ctivity against Macrophage-tropic viruses, MBP is therefore believed to offer similar anti-HCV activity against T cell-tropic viruses.

Results in Examples 1-2 taught that the anti-HIV agent of the present invention offer an anti-HIV activity against CCR5-tropic viruses, CXCR4-tropic viruses and CCR5/CXCR4-tropic viruses. It was then also revealed that the anti-HIV agent of the present invention offers an anti-HIV activity against Macrophage-tropic viruses, T cell-tropic viruses and Macrophage/T cell-tropic viruses.

### [Industrial Applicability]

The anti-HIV agent according to the present invention containing MBP as an active component offers neutralizing activity against various HIV strains including the recombinant epidemic strain CRF01_AE virus which is regarded as the most difficult strain to neutralize its activity. In other words, the anti-HIV agent of the present invention offers necessary anti-HIV activity, irrespective of viral Subtypes and kind/sort of chemokine receptor-tropism and Macrophage/T cell-tropism, against Subtype B HIV, Subtype D HIV and CRF01_AE which are terrible hard to neutralize at this moment.

Then, the anti-HIV agent according to the present invention also offers an anti-HIV activity against CCR5-tropic viruses, CXCR4-tropic viruses and CCR5/CXCR4-tropic viruses. Further, the anti-HIV agent according to the present invention also offers an anti-HCV activity against Macrophage-tropic viruses, T cell-tropic viruses and Macrophage/T cell-tropic viruses.

Because the anti-HIV agent according to the present invention targeted to a carbohydrate chain, MBP resistant strain due to HIV genomic mutation would not likely to appear. Further, because MBP itself stays inherently in a living body, any adverse effect due to the compounds utilized in the conventional HIV therapies would not be expected.

Accordingly, the anti-HIV agent of the present invention would treat various aspects of infectious diseases due to HIV and is significantly useful on the therapy for such diseases.

## Claims

1. An anti-HIV agent which comprises a mannose binding protein (MBP) as an active component.

2. The anti-HIV agent according to claim 1 wherein said MBP has HIV proliferation suppressive activity.

3. The anti-HIV agent according to claim 2 wherein said proliferation suppressive activity is HIV neutralizing activity.

4. The anti-HIV agent according to claim 2 wherein said proliferation suppressive activity is HIV budding suppressive activity.

5. The anti-HIV agent according to claim 1 or 2 wherein said MBP is isolated and purified from a human serum.

6. The anti-HIV agent according to claim 1 or 2 wherein said MBP is secreted by an animal cell following gene expression.

7. The anti-HIV agent according to claim 6 wherein said animal cell is Chinese Hamster Ovary cell.

8. The anti-HIV agent according to claim 1 or 2 wherein said HIV is an HIV strain belonging to Subtype B of Group M of HIV Type 1.

9. The anti-HIV agent according to claim 1 or 2 wherein said HIV is an HIV strain belonging to Subtype D of Group M of HIV Type 1.

10. The anti-HIV agent according to claim 1 or 2 wherein said HIV is a recombinant epidemic strain.

11. The anti-HIV agent according to claim 10 wherein said recombinant epidemic strain is CRF01_AE.

12. The anti-HIV agent according to claim 1 or 2 wherein said HIV is a virus having tropism towards CCR5.

13. The anti-HIV agent according to claim 1 or 2 wherein said HIV is a virus having tropism towards CXCR4.

14. The anti-HIV agent according to claim 1 or 2 wherein said HIV is a virus having tropism towards both CCR5 and CXCR4.

15. The anti-HIV agent according to claim 1 or 2 wherein said HIV is a virus having tropism towards macrophages.

16. The anti-HIV agent according to claim 1 or 2 wherein said HIV is a virus having tropism towards T cell.

17. The anti-HIV agent according to claim 1 or 2 wherein said HIV is a virus having tropism towards both macrophages and T cell.

18. A method for evaluating an anti-HIV activity of MBP, the method comprising the steps of:
(1) culturing HIV infected cells prepared by exposing target cells to HIV;
(2) preparing clean cells by washing the infected cells;
(3) culturing the clean cells under the presence of MBP; and
(4) detecting p24 protein from HIV in the culture supernatant.

19. A method for evaluating an anti-HIV activity of MBP, the method comprising the steps of:
(a) culturing a first mixed system including HIV and MBP;
(b) culturing a second mixed system including target cells and MBP;
(c) preparing infected cells by combining said first mixed system and second mixed system;
(d) culturing the infected cells;
(e) preparing clean cells by washing the infected cells;
(f) culturing the clean cells; and
(g) detecting p24 protein from HIV in the culture supernatant.

20. The method according to claim 19 wherein said steps (a) and (b) are performed in parallel.

21. The method according to claim 19 or 20 wherein the clean cells are washed in said step (f) in the presence of MBP.

22. The method according to claim 18 or 19 wherein said anti-HIV activity is HIV proliferation suppressive activity.

23. The method according to claim 22 wherein said proliferation suppressive activity is HIV neutralizing activity.

24. The method according to claim 22 wherein said proliferation suppressive activity is HIV budding suppressive activity.

25. The method according to claim 18 or 19 wherein said MBP is isolated and purified from a human serum.

26. The method according to claim 18 or 19 wherein said MBP is secreted by an animal cell following gene expression.

27. The method according to claim 26 wherein said animal cell is Chinese Hamster Ovary cell.

28. The method according to claim 18 or 19 wherein said HIV is an HIV strain belonging to Subtype B of Group M of HIV Type 1.

29. The method according to claim 18 or 19 wherein said HIV is an HIV strain belonging to Subtype D of Group M of HIV Type 1.

30. The method according to claim 18 or 19 wherein said HIV is a recombinant epidemic strain.

31. The method according to claim 30 wherein said recombinant epidemic strain is CRF01_AE.

32. The method according to claim 18 or 19 wherein said HIV is a virus having tropism towards CCR5.

33. The method according to claim 18 or 19 wherein said HIV is a virus having tropism towards CXCR4.

34. The method according to claim 18 or 19 wherein said HIV is a virus having tropism towards both CCR5 and CXCR4.

35. The method according to claim 18 or 19 wherein said HIV is a virus having tropism towards macrophages.

36. The method according to claim 18 or 19 wherein said HIV is a virus having tropism towards T cells.

37. The method of the evaluation according to claim 18 or 19 wherein said HIV is a virus having tropism towards both macrophages and T cells.

38. MBP possessing an anti-HIV activity as determined by the method according to claim 18 or 19.

39. Use of an anti-HIV agent containing MBP as an active component for an HIV-infected individual.

40. Use of an anti-HIV agent according to claim 39 wherein said HIV-infected individual is a patient with a virus having tropism towards CCR5.
